# EUROPÄISCHE PATENTANMELDUNG

(11) **EP 0 736 513 A1**
(43) Veröffentlichungstag der Anmeldung: **09.10.1996**
(21) Anmeldenummer: 96104487.2
(22) Anmeldetag: 21.03.1996
(51) Int. Cl.: C07C 69/75, C09K 19/30, C09K 19/34

(54) **1-Fluorcyclohexen-Difluorphenyl-Derivate**

(30) Priorität: 04.04.1995 CH 948/95
(71) Anmelder: F. HOFFMANN-LA ROCHE AG, 4070 Basel (CH)
(72) Erfinder: Buchecker, Richard, 8008 Zürich (CH); Marck, Guy, 68170 Rixheim (FR)
(74) Vertreter: Kjellsaa-Berger, Hanny, Dr.

(57) **Zusammenfassung**

Die vorliegende Erfindung betrifft Verbindungen der allgemeinen Formel 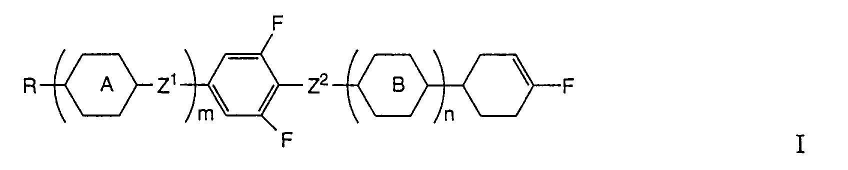 worin
- R: Alkyl oder Alkenyl mit höchstens 12 Kohlenstoffatomen;
- Ringe A und B: unabhängig voneinander 1,4-Phenylen, trans-1,4-Cyclo-hexylen oder trans-1,3-Dioxan-2,5-diyl;
- Z¹: eine Einfachbindung oder -CH₂CH₂-; und
- Z²: eine Einfachbindung, -CH₂CH₂-, -OCH₂-, -C≡C-, -COO- oder -OOC- bedeuten; und
- m, n: 0 oder 1 sind, mit der Massgabe, dass m + n ≦ 1 ist;
sowie flüssigkristalline Gemische, die solche Verbindungen enthalten und die Verwendung solcher Verbindungen bzw. Gemische für elektro-optische Anzeigevorrichtungen.

## Beschreibung

Die vorliegende Erfindung betrifft neue flüssigkristalline 1-Fluorcyclo-hexen-Difluorphenyl-Derivate, sowie flüssigkristalline Mischungen, welche solche Verbindungen enthalten und die Verwendung solcher Verbindungen und Mischungen in elektro-optischen Vorrichtungen.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten Zellen, besonders die aktiv angesteuerten, z.B. TFT-Zellen ("thin film transistor") wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), aber trotzdem ausreichend niedere Viskosität besitzen und in den Zellen kurze Ansprechzeiten, tiefe Schwellenspannungen und einen hohen Kontrast ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine möglichst hohe positive dielektrische Anisotropie und gleichzeitig eine möglichst geringe Leitfähigkeit aufweisen. Diese letztere Eigenschaft ist vorallem für TFT-Zellen von besonderer Wichtigkeit. Leider führen aber Komponenten mit höherer dielektrischer Anisotropie infolge ihres besseren Lösungsvermögens für ionische Verunreinigungen meist zu einer erhöhten Leitfähigkeit in Mischungen. Es werden daher Komponenten gesucht, die sich durch eine möglichst hohe dielektrische Anisotropie bei gleichzeitig möglichst geringer Leitfähigkeit auszeichnen.

Die erfindungsgemässen Verbindungen, welche sich alle durch die Kombination eines 1,3-Difluor-1,4-phenylen Ringes mit einem 4-Fluor-cyclo-hexen-3-yl Ring auszeichnen, weisen alle, trotz niedriger Polarität, eine niedrige Schwellenspannung (V₁₀) auf und sind daher insbesondere für die Anwendung in TFT-Zellen geeignet.

Es sind dies Verbindungen der Formel 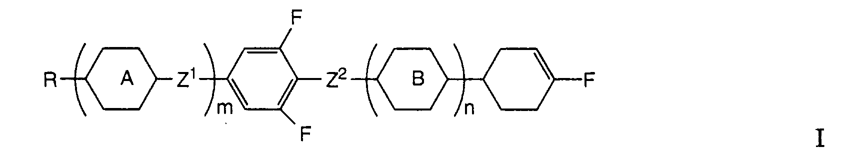 worin
- R: Alkyl oder Alkenyl mit höchstens 12 Kohlenstoffatomen;
- Ringe A und B: unabhängig voneinander 1,4-Phenylen, trans-1,4-Cyclohexylen oder trans-1,3-Dioxan-2,5-diyl;
- Z¹: eine Einfachbindung oder -CH₂CH₂-; und
- Z²: eine Einfachbindung, -CH₂CH₂-, -OCH₂-, -C≡C-, -COO-oder -OOC- bedeuten; und
- m, n: 0 oder 1 sind, mit der Massgabe, dass m + n ≦ 1 ist.

Der Ausdruck "Alkyl oder Alkenyl mit höchstens 12 Kohlenstoffatomen" bedeutet im Rahmen der vorliegenden Erfindung geradkettige oder verzweigte (gegebenenfalls chirale) Alkylreste, geradkettige 3E-Alkenyl- oder Alkenylreste mit endständiger Doppelbindung oder an einem gesättigten Ring wie trans-1,4-Cyclohexylen oder trans-1,3-Dioxan-2,5-diyl auch 1E-Alkenylreste. Diese Reste haben höchstens 12, vorzugsweise jedoch höchstens 7 Kohlenstoffatome. Es sind dies beispielsweise Alkylreste, wie Methyl, Ethyl, Propyl, Butyl, Pentyl, Hexyl, Heptyl, Isopropyl, 2-Methyl-butyl, 2-Methyl-pentyl oder 2-Methyl-hexyl; 1E-Alkenylreste, wie 1E-Propenyl, 1E-Butenyl, 1E-Pentenyl, 1E-Hexenyl oder 1E-Heptenyl; 3E-Alkenylreste, wie 3E-Pentenyl, 3E-Hexenyl oder 3E-Heptenyl; oder Alkenylreste mit endständiger Doppelbindung, wie Vinyl, 3-Butenyl, 4-Pentenyl, 5-Hexenyl oder 6-Heptenyl.

Bevorzugt sind Verbindungen der Formeln 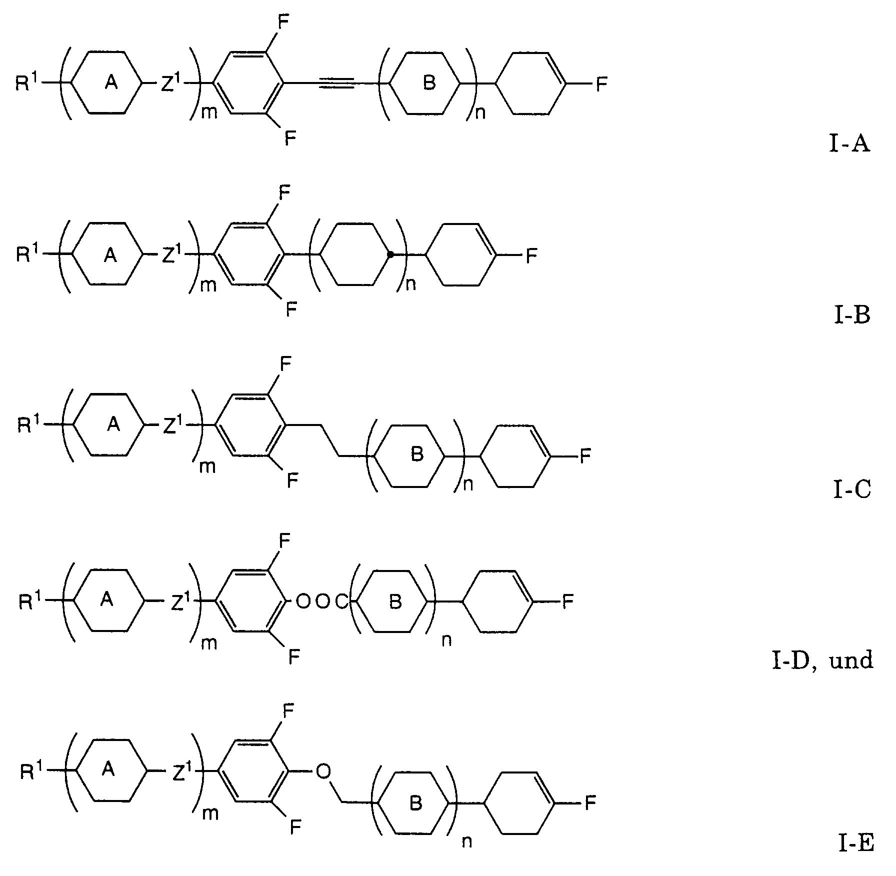 worin A, Z¹, B, m und n wie oben definiert sind; und R¹ Alkyl, 3E-Alkenyl oder an einem gesättigten Ring auch Vinyl oder 1E-Alkenyl mit höchstens 7 Kohlenstoffatomen bedeutet.

Ganz besonders bevorzugte Verbindungen der Formel I-A sind diejenigen, worin Z¹ eine Einfachbindung bedeutet, sowie Verbindungen, worin der Ring B trans-1,4-Cyclohexylen bedeutet, d.h. Verbindungen der Formeln 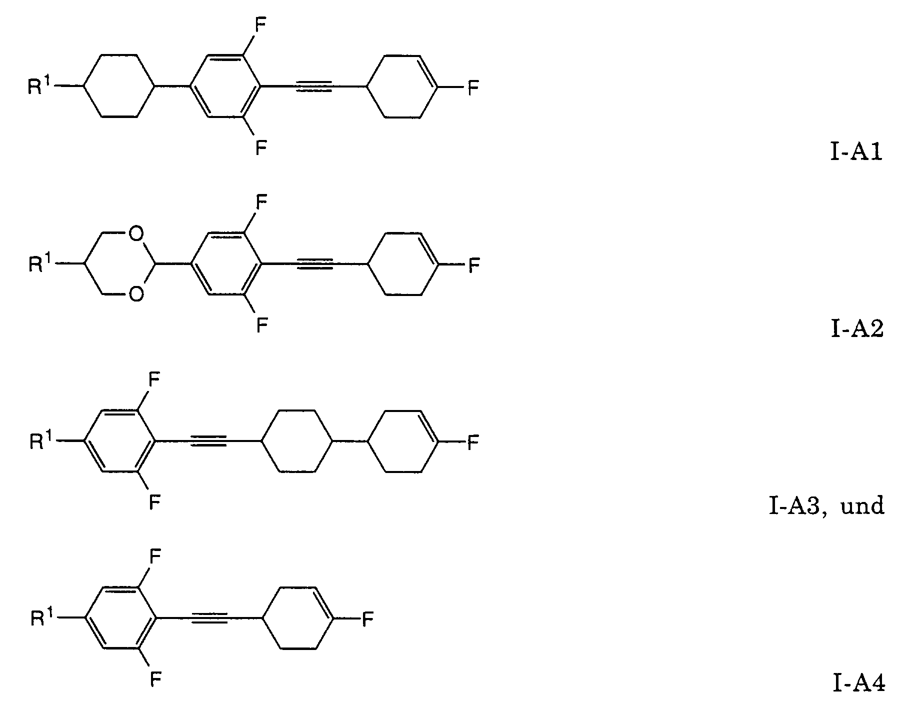 worin R¹ Alkyl, 3E-Alkenyl oder an einem gesättigten Ring auch Vinyl oder 1E-Alkenyl mit höchstens 7 Kohlenstoffatomen bedeutet. Besonders bevorzugte Verbindungen der Formel I-B sind 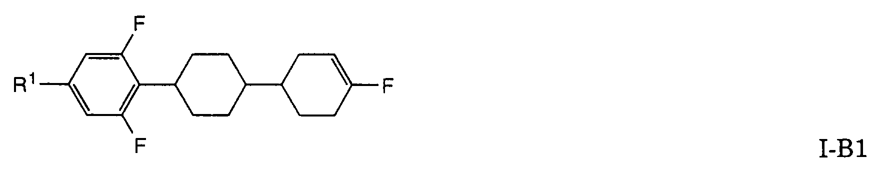 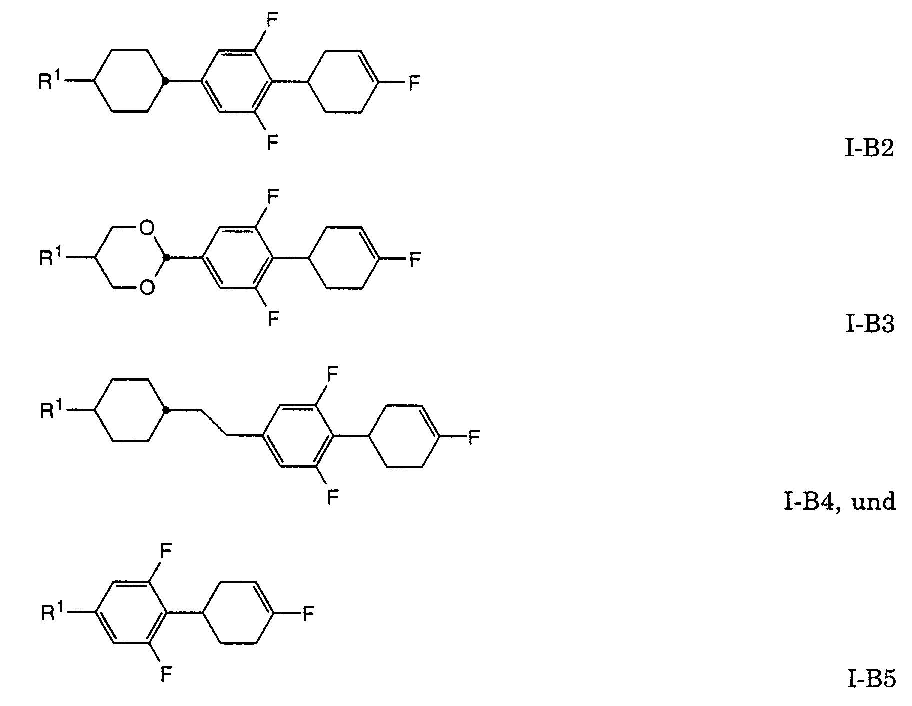 worin R¹ Alkyl, 3E-Alkenyl oder an einem gesättigten Ring auch Vinyl oder 1E-Alkenyl mit höchstens 7 Kohlenstoffatomen bedeutet.

Besonders bevorzugte Verbindungen der Formel I-C sind diejenigen, worin Z¹ eine Einfachbindung bedeutet, d.h. Verbindungen der Formeln 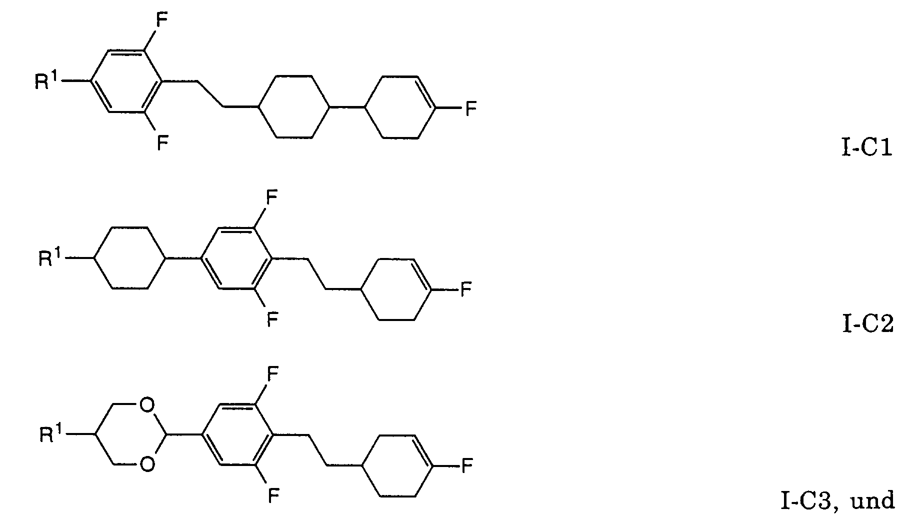 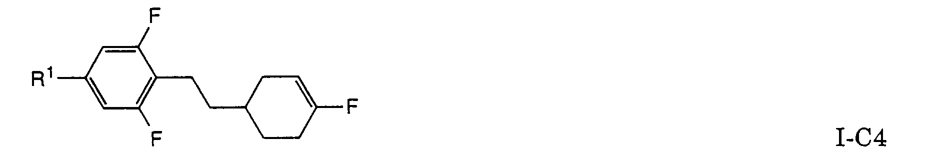 worin R¹ Alkyl, 3E-Alkenyl oder an einem gesättigten Ring auch Vinyl oder 1E-Alkenyl mit höchstens 7 Kohlenstoffatomen bedeutet. Besonders bevorzugte Verbindungen der Formel I-D sind 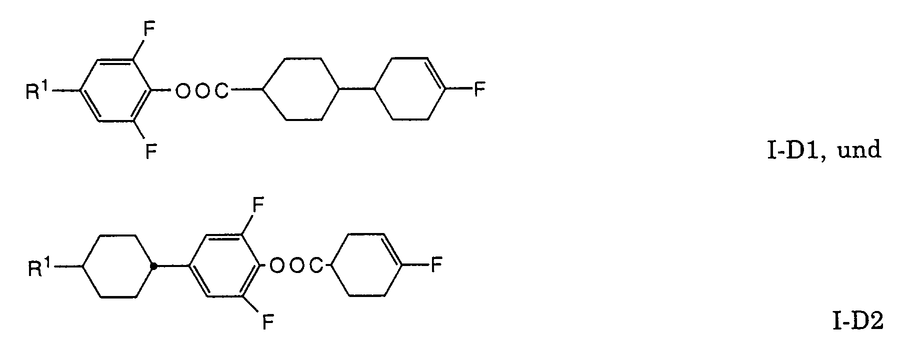 worin R¹ Alkyl, 3E-Alkenyl oder an einem gesättigten Ring auch Vinyl oder 1E-Alkenyl mit höchstens 7 Kohlenstoffatomen bedeutet. Besonders bevorzugte Verbindungen der Formel I-E sind 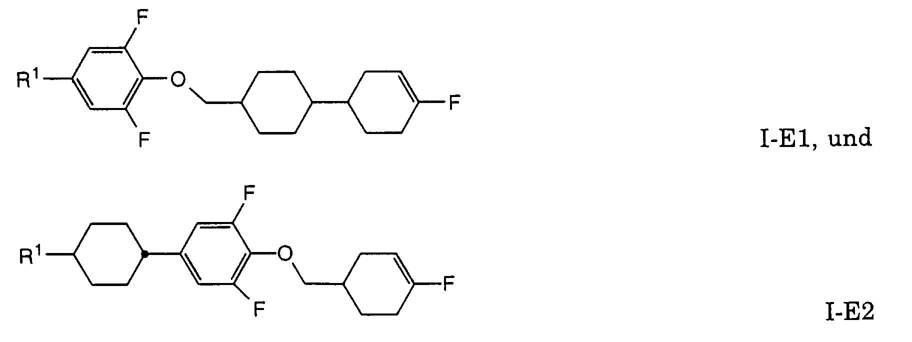 worin R¹ Alkyl, 3E-Alkenyl oder an einem gesättigten Ring auch Vinyl oder 1E-Alkenyl mit höchstens 7 Kohlenstoffatomen bedeutet.

Die Verbindungen der Formel I können in an sich bekannter Weise hergestellt werden. So können beispielsweise Verbindungen der Formel I-A aus einem entsprechend substituierten Cyclohexanon (**1**) hergestellt werden. Dabei wird das Cyclohexanon (**1**) analog zu einem in US Patent 4 212 815 oder in Organic Reactions 35, 531 (1988) beschriebenen Verfahren mit Diethylaminoschwefeltrifluorid (DAST) in das entsprechende 1-Fluorcyclohexen (**2**) übergeführt. Die Reaktion wird vorzugsweise in polaren aprotischen und inerten Lösungsmitteln, wie Dimethoxyethan, Diglyme, Dioxan und dergleichen bei Temperaturen von ca. 20°C bis ca. 50°C durchgeführt. Die Entstehung von geminalen Difluorcyclohexan Derivaten kann durch Zugabe einer katalytischen Menge von Säure wie Oleum oder Trifluormethansulfonsäure unterdrückt werden. Die weiteren Reaktionsschritte, ausgehend von **2**, welche zu den Acetylenen der Formel I-A führen, sind unter anderem auch in EP 593 997 beschrieben worden und in Schema 1 aufgezeichnet.

Die Verbindungen der Formel I-B, worin n = 0 ist, können ausgehend von einem entsprechend substituierten Difluorphenyl (**7**), welches in Gegenwart von Butyllithium mit 1,4-Dioxa-spiro[4.5]decan-8-on (**8**) umgesetzt wird, hergestellt werden; die Verbindungen der Formel I-B, worin n = 1 ist, können durch Umsetzen des entsprechend substituierten Difluorphenyls (**7**) mit 4-(1,4-Dioxa-spiro[4.5]dec-8-yl)-cyclohexanon (**9**) hergestellt werden, wie dies in Schema 2 aufgezeichnet ist.

Verbindungen der Formel I-C können beispielsweise analog zu den Verbindungen der Formel I-A hergestellt werden, dabei wird jedoch zweckmässig die Ketogruppe von **1** geschützt und erst nach der Reduktion der Dreifachbindung (**15** → **16**) wieder freigesetzt und anschliessend fluoriert, siehe Schema 3.

Die Herstellung von Verbindungen der Formel I-D und I-E kann auf an sich bekannte Weise, wie beispielsweise im Schema 4 aufgezeichnet, erfolgen.

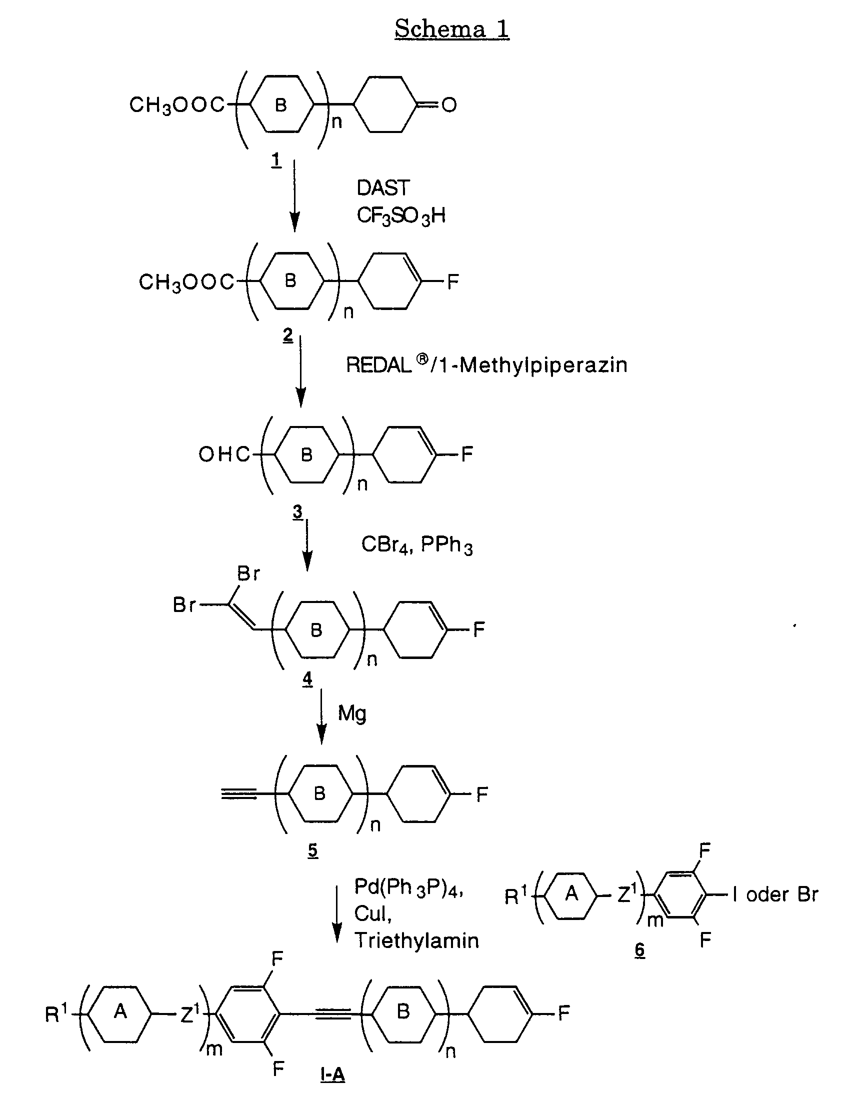

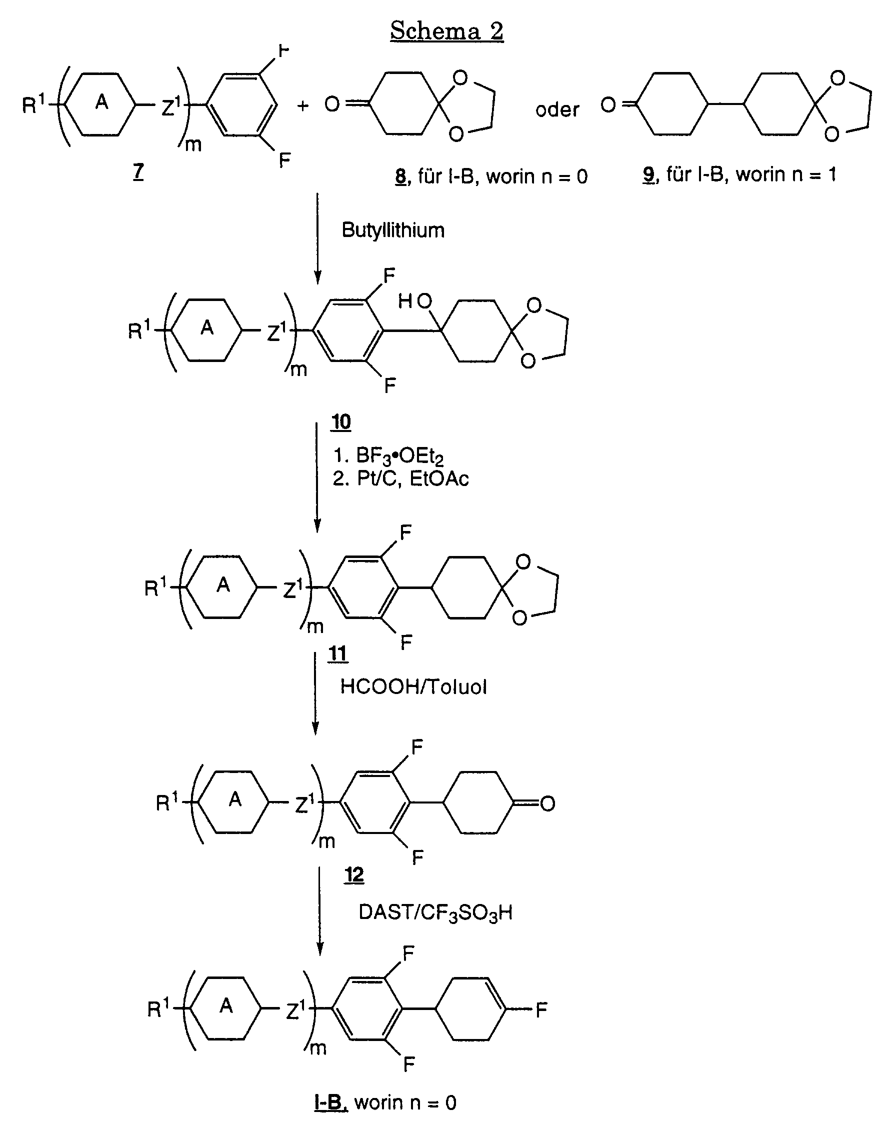

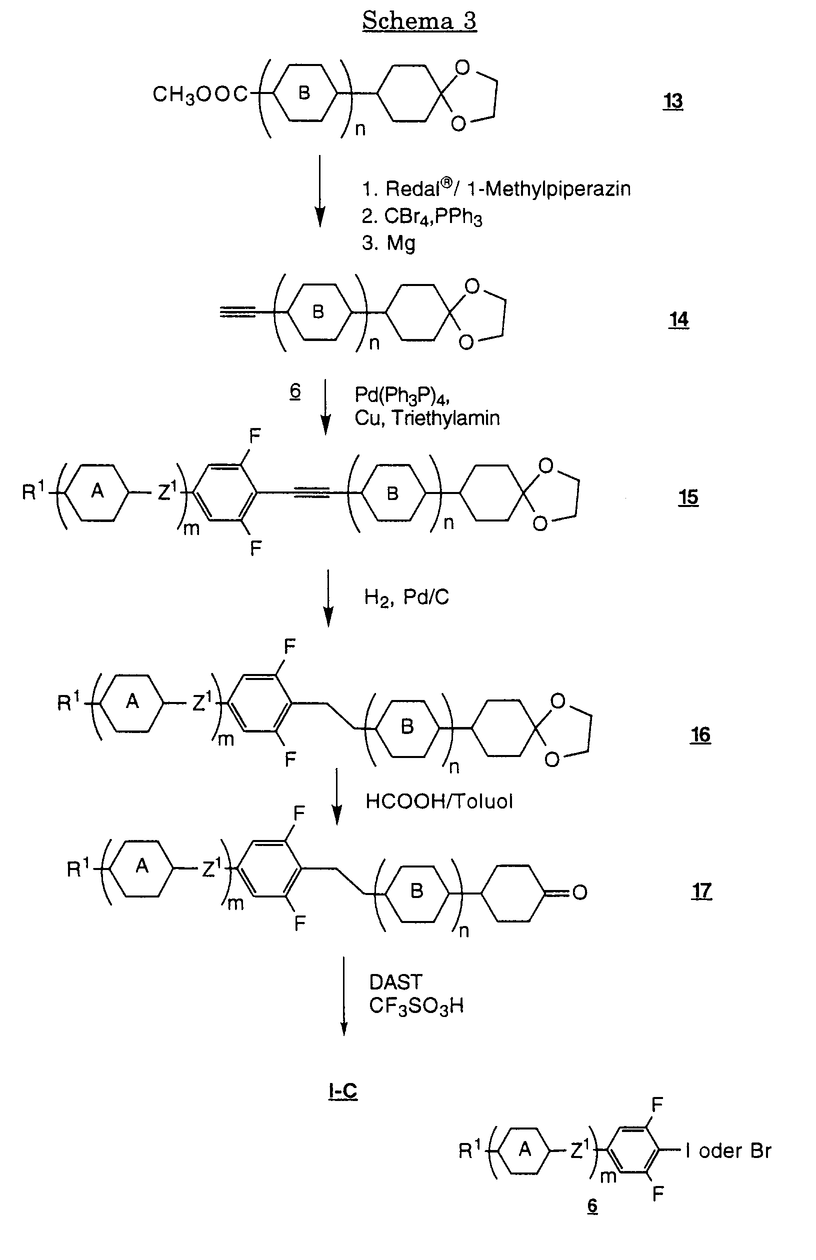

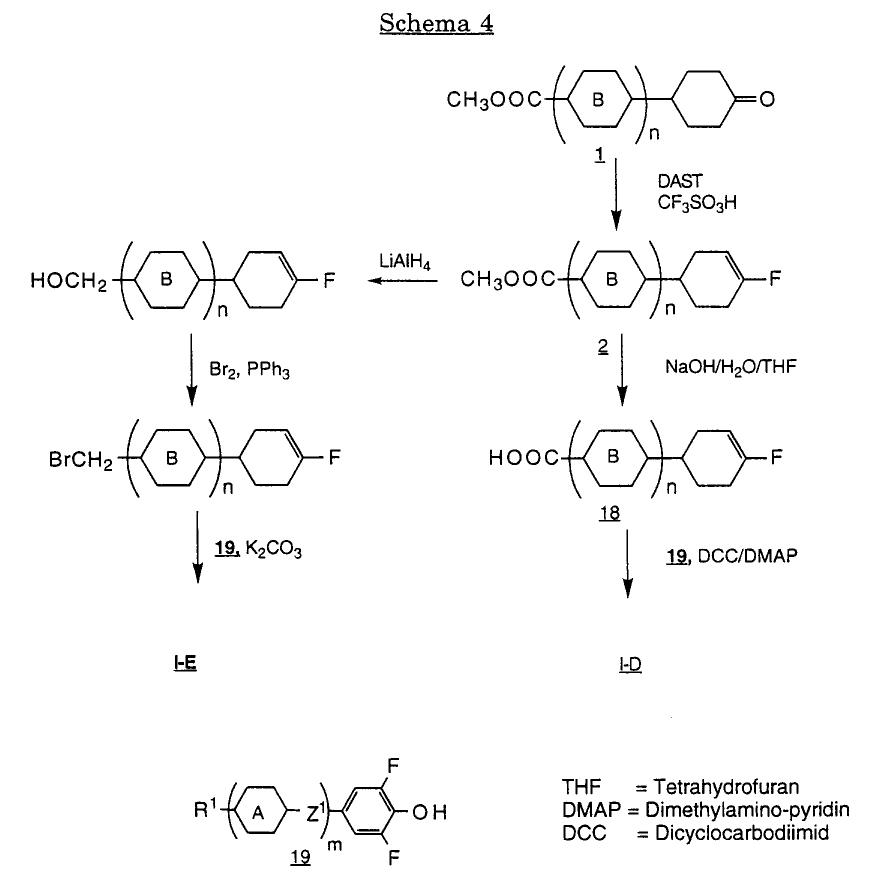

Die in den Schemata verwendeten Symbole sind wie weiter oben definiert.

Die Verbindungen der Formel I können in Form von Gemischen untereinander und/oder mit anderen Flüssigkristallkomponenten verwendet werden. Die Erfindung betrifft daher ebenfalls flüssigkristalline Gemische mit mindestens 2 Komponenten, welche dadurch gekennzeichnet sind, dass mindestens eine Komponente eine Verbindung der Formel I ist. Eine zweite und gegebenenfalls weitere Komponenten können weitere Verbindungen der allgemeinen Formel I oder andere geeignete Flüssigkristallkomponenten sein. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, viele davon sind zudem im Handel erhältlich.

Aufgrund der guten Löslichkeit der erfindungsgemässen Verbindungen der Formel I in anderen Flüssigkristallmaterialien und aufgrund ihrer guten Mischbarkeit untereinander kann der Anteil an Verbindungen der Formel I in den erfindungsgemässen Gemischen relativ hoch sein und beispielsweise 1-70 Gew.-% betragen. Im allgemeinen ist ein Anteil von etwa 3-40 Gew.-%, insbesondere von etwa 5-20 Gew.-%, an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 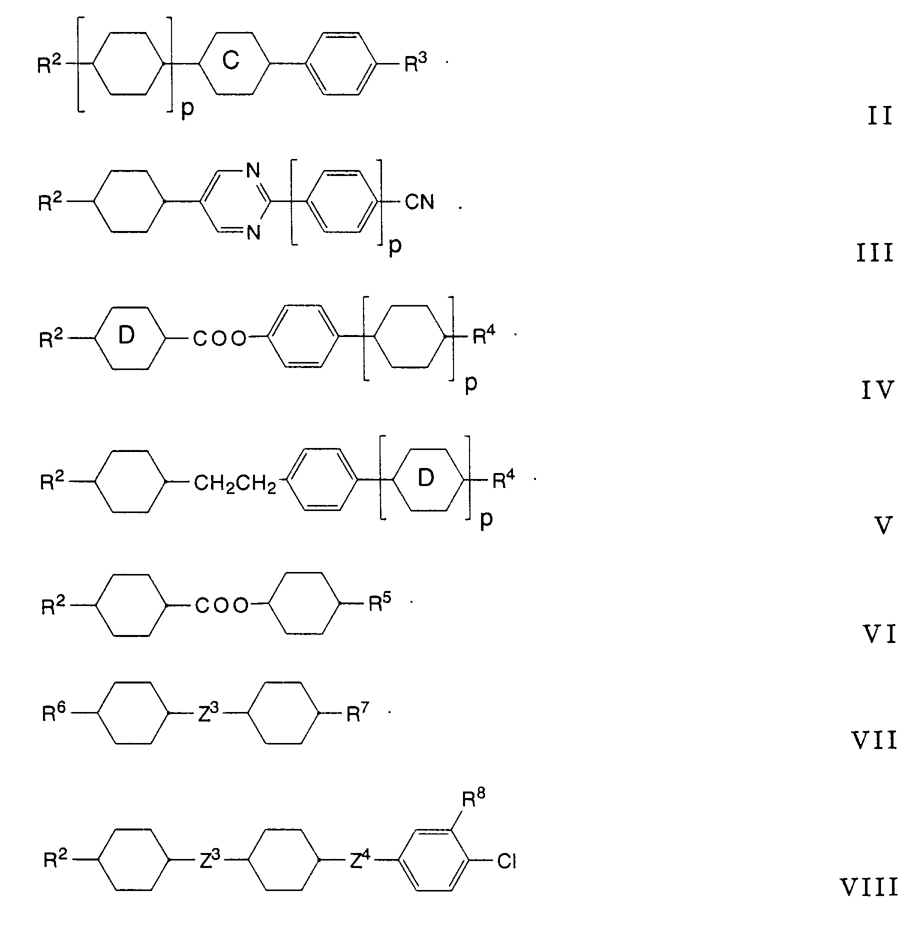 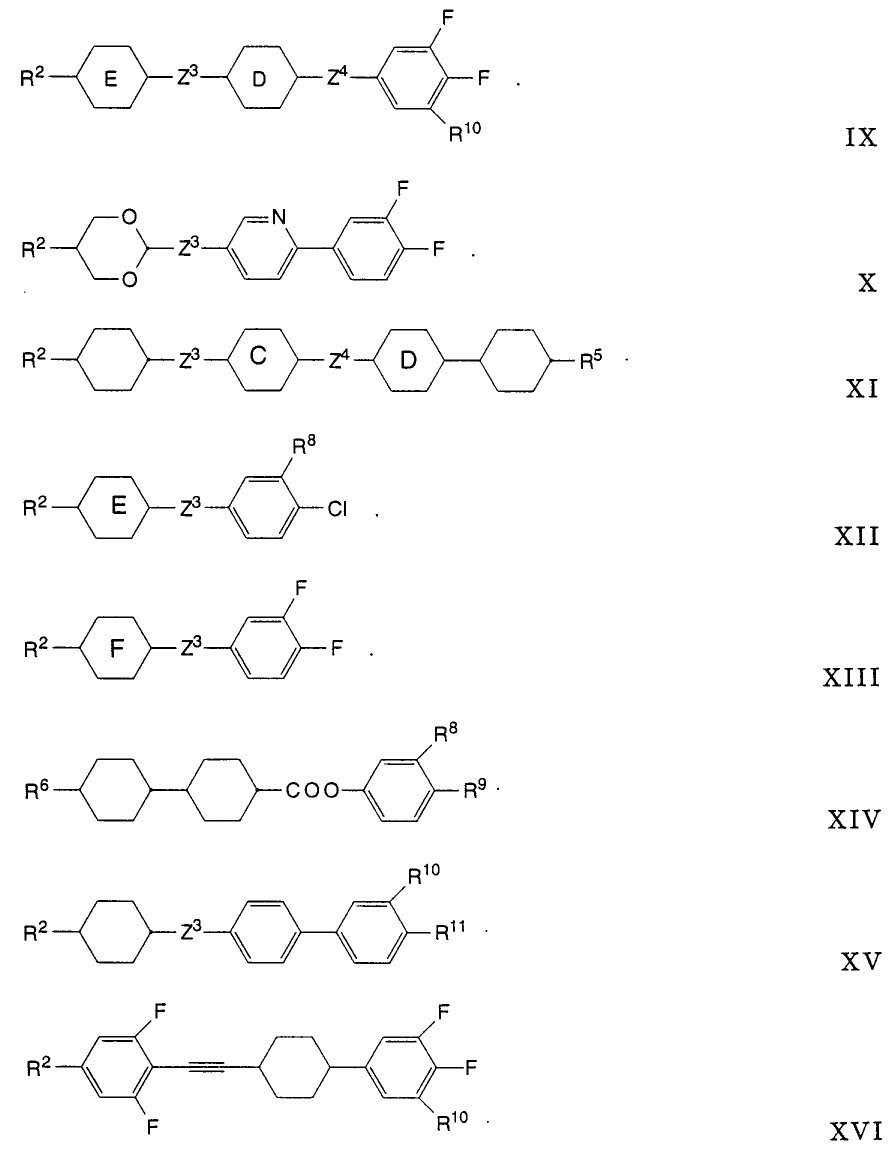 worin
- R²,R⁵: Alkyl, Alkoxyalkyl, 3E-Alkenyl, 4-Alkenyl oder an einem gesättigten Ring auch 1E-Alkenyl;
- p: 0 oder 1;
- Ring C: 1,4-Phenylen, Pyridin-2,5-diyl, Pyrimidin-2,5-diyl, trans-1,4-Cyclohexylen oder trans-1,3-Dioxan-2,5-diyl;
- R³: Cyano, Isothiocyanato, Fluor, Alkyl, 3E-Alkenyl, 4-Alkenyl, Alkoxy, 2E-Alkenyloxy, 3-Alkenyloxy oder 1-Alkinyl;
- Ring D: 1,4-Phenylen oder trans-1,4-Cyclohexylen;
- R⁴: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy;
- R⁶: Alkyl, 1E-Alkenyl, 3E-Alkenyl oder 4-Alkenyl;
- R⁷: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl;
- Z³, Z⁴: eine Einfachbindung oder -CH₂CH₂-, wobei zwei aromatische Ringe immer durch eine Einfachbindung verknüpft sind;
- Ring E: trans-1,4-Cyclohexylen oder trans-1,3-Dioxan-2,5-diyl;
- R⁸: Wasserstoff, Fluor oder Chlor;
- R⁹: Cyano, Fluor oder Chlor;
- R¹⁰: Wasserstoff oder Fluor;
- R¹¹: Fluor oder Chlor; und
- Ring F: Pyrimidin-2,5-diyl, trans-1,4-Cyclohexylen oder trans-1,3-Dioxan-2,5-diyl bedeuten.

Die im Zusammenhang mit den Verbindungen der Formeln II bis XVI verwendeten Ausdrücke Alkyl, 1E-Alkenyl, 3E-Alkenyl und Alkenyl mit endständiger Doppelbindung sind weiter oben definiert;
"4-Alkenyl" bedeutet vorzugsweise geradkettige Alkenylreste mit höchstens 12 Kohlenstoffatomen, in denen sich die Doppelbindung in 4-Stellung befindet, wie beispielsweise 4-Pentenyl, 4-Hexenyl oder 4-Heptenyl.
"Aromatische Ringe" bedeutet Ringe, wie beispielsweise 1,4-Phenylen, Pyridin-2,5-diyl oder Pyrimidin-2,5-diyl.
"Gesättigte Ringe" bedeutet trans-1,4-Cyclohexylen oder trans-1,3-Dioxan-2,5-diyl.
"Alkyloxyalkyl" bedeutet vorzugsweise geradkettige Reste mit höchstens 12 Kohlenstoffatomen, wie beispielsweise Methoxymethyl, Ethoxymethyl, Propyloxymethyl, Butyloxymethyl und dergleichen.
"Alkyloxy" bedeutet vorzugsweise geradkettige Reste mit höchstens 12 Kohlenstoffatomen, wie beispielsweise Methoxy, Ethoxy, Propyloxy, Butyloxy, Pentyloxy, Hexyloxy, Heptyloxy und dergleichen.
"2E- bzw. 3-Alkenyloxy" bedeutet vorzugsweise geradkettige Alkenyloxyreste mit höchstens 12 Kohlenwasserstoffatomen, in denen sich die Doppelbindung in 2- bzw 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3-Pentenyloxy, 3-Hexenyloxy, 3-Heptenyloxy, 4-Pentenyloxy, 5-Hexenyloxy, 6-Heptenyloxy und dergleichen.
"1-Alkinyl" bedeutet vorzugsweise geradkettige Alkinylreste mit höchstens 12 Kohlenwasserstoffatomen, in denen die Dreifachbindung sich in 1-Stellung befindet, wie beispielsweise Ethinyl, 1-Propinyl, 1-Butinyl, 1-Pentinyl und dergleichen.

Die erfindungsgemässen Mischungen können ferner optisch aktive Verbindungen (z.B. optisch aktive 4'-Alkyl- oder 4'-Alkoxy-4-biphenyl-carbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe der Helix, Farbe, Extinktion und dergleichen bestimmt. Im allgemeinen beträgt der Anteil optisch aktiver Verbindungen und dichroitischer Farbstoffe höchstens je etwa 10 Gew.% im Gesamtgemisch.

Die Herstellung der flüssigkristallinen Gemische und der elektrooptischen Vorrichtung kann in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische Phase, N eine nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit und Δn die optische Anisotropie.

### Beispiel 1

a) In einem getrockneten und mit Stickstoff begasten Kolben wurden bei Raumtemperatur zu 5,0 g 4-(trans-4-Methoxycarbonylcyclohexyl)cyclo-hexanon zunächst 50 ml Dimethoxyethan und anschliessend 200 µl Trifluormethansulfonsäure zugegeben. Nach 5 Minuten bei Raumtemperatur wurden 2,82 ml Dimethylaminoschwefeltrifluorid zugefügt. Die Reaktionslösung wurde nach 20 Stunden Rühren bei Raumtemperatur zwischen gesättigter NaHCO₃-Lösung und Methylenchlorid verteilt. Die organische Phase wurde abgetrennt, mit einer Natriumcarbonatlösung und dann mit Wasser gewaschen. Die wässrigen Phasen wurden einzeln je zweimal mit Methylenchlorid extrahiert. Die organischen Phasen wurden vereinigt, über Magnesiumsulfat getrocknet, filtriert, und das Filtrat eingedampft. Das Rohprodukt (7,25 g) wurde chromatographisch an 250 g Kieselgel gereinigt. Laufmittel: Toluol. Dies ergab 1,46 g 1-Fluor-4-(trans-4-methoxycarbonylcyclohexyl)cyclohex-1-en.
b) In einem Sulfierkolben unter Stickstoffbegasung wurden 0,84 g 1-Fluor-4-(trans-4-methoxycarbonylcyclohexyl)cyclohex-1-en zu einer Lösung von 0,7 g Natriumhydroxid in 10 ml Wasser und 20 ml Tetrahydrofuran gegeben und 20 Stunden bei 60°C gerührt. Die Reaktionslösung wurde zu 2N Salzsäure gegossen und mit Methylenchlorid extrahiert. Die organische Phase wurde zweimal mit Wasser gewaschen. Die wässrigen Phasen wurden einzeln zweimal mit Methylenchlorid extrahiert. Die organischen Phasen wurden vereinigt, über Magnesiumsulfat getrocknet, filtriert und das Filtrat eingedampft. Dies ergab 0,79 g weisses 1-Fluor-4-(trans-4-carboxycyclohexyl)cyclohex-1-en, welches ohne weitere Reinigung in der nächsten Stufe eingesetzt wurde.
c) Das Rohprodukt aus b), 0,79 g 1-Fluor-4-(trans-4-carboxycyclohexyl)-cyclohex-1-en, wurde unter Stickstoff zusammen mit 0,66 g 2,6-Difluor-4-propylphenol und 60 mg Dimethylaminopyridin in 10 ml Methylenchlorid auf 0°C gekühlt und innert 45 Minuten mit einer Lösung von 1,0 g Dicyclohexylcarbodiimid in 5 ml Methylenchlorid versetzt. Nach 30 Minuten Rühren bei Raumtemperatur wurde die weisse Suspension über ein Celite/Kieselgelpolster filtriert. Das Filtrat wurde vollständig eingedampft und ergab 1,5 g trans-4-(1-Fluorcyclohex-1-en-4-yl)cyclohexancarbonsäure-2,6-difluor-4-propylphenylester, welches durch mehrmaliges Umkristallisieren aus Hexan 0,62 g weisse Kristalle ergab. Smp. (C/N) 58,4°C, Klp. (N/I) 96,1°C.

Auf analoge Weise können folgende Verbindungen hergestellt werden:
trans-4-(1-Fluorcyclohex-1-en-4-yl)cyclohexancarbonsäure-2,6-difluor-4-pentylphenylester;
trans-4-(1-Fluorcyclohex-1-en-4-yl)cyclohexancarbonsäure-2,6-difluor-4-(3-butenyl)phenylester;
4-Fluorcyclohex-3-encarbonsäure-2,6-difluor-4-(trans-4-propylcyclohexyl)-phenylester;
4-Fluorcyclohex-3-encarbonsäure-2,6-difluor-4-(trans-4-pentylcyclohexyl)-phenylester.

### Beispiel 2

a) Unter Stickstoffbegasung wurden 3,9 g Tetrabrommethan in 30 ml Methylenchlorid gelöst, auf 0°C gekühlt und innert 15 Minuten mit einer Lösung von 6,2 g Triphenylphosphin in 15 ml Methylenchlorid versetzt. Die Lösung wurde bei 0°C gerührt und anschliessend bei 0°C innert 15 Minuten mit einer Lösung von 1,25 g trans-4-(1-Fluorcyclohexen-4-yl)cyclohexancarboxaldehyd in 8 ml Methylenchlorid versetzt. Nach 1 Stunde bei 0°C wurde die orange Reaktionslösung auf 100 ml Pentan gegossen und die Suspension filtriert. Das Filtrat ergab nach Eindampfen 3,0 g Rohprodukt als farblose Flüssigkeit. Dieses wurde in Cyclohexan aufgenommen und bei 50°C innert 15 Minuten mit 5 ml Wasserstoffperoxid 30%ig versetzt. Die Suspension wurde dreimal mit Methanol/ Wasser 3:2 extrahiert. Die wässrigen Phasen wurden einzeln je zweimal mit Cyclohexan extrahiert. Die organischen Phasen wurden vereinigt, über Magnesiumsulfat getrocknet, filtriert und das Filtrat eingedampft. Dies ergab 1,89 g farbloses trans-1-(2,2-Dibromvinyl)-4-(1-fluorcyclohex-1-en-4-yl)cyclohexan.
b) Unter Stickstoffbegasung wurden 0,138 g Mg-Späne getrocknet, mit 2 ml trockenem Tetrahydrofuran überschichtet, auf 60°C erwärmt und mit 2 Iodkristallen aktiviert. Innert 30 Minuten wurde eine Lösung von 1,89 g trans-1-(2,2-Dibromvinyl)-4-(1-fluorcyclohex-1-en-4-yl)cyclohexan in 20 ml Tetrahydrofuran zugetropft. Nach 2 Stunden bei 60°C wurde auf Raumtemperatur gekühlt und das Reaktionsgemisch zwischen Wasser und Ether verteilt. Die organische Phase wurde zweimal mit Wasser gewaschen. Die wässrigen Phasen wurden einzeln je zweimal mit Ether extrahiert. Die organischen Phasen wurden vereinigt, über Magnesiumsulfat getrocknet, filtriert und das Filtrat vollständig eingedampft. Das Rohprodukt wurde an Kieselgel mit Cyclohexan gereinigt und ergab 0,83 g trans-1-Ethinyl-4-(1-fluorcyclohex-1-en-4-yl)cyclohexan.
c) Unter Stickstoffbegasung wurden 0,8 g trans-1-Ethinyl-4-(1-fluorcyclo-hex-1-en-4-yl)cyclohexan, 1,0 g 1-Iod-2,6-difluor-4-propylbenzol in 10 ml Triethylamin vorgelegt und mit 0,128 g Tetrakis(triphenylphosphin)palladium(0) und 14 mg Kupfer(I)iodid versetzt. Die Reaktion wurde 17 Stunden am Rückfluss gehalten. Nach dem Abkühlen wurde das Reaktionsgemisch zwischen Wasser und Ether verteilt. Die organische Phase wurde zweimal mit Wasser gewaschen. Die wässrigen Phasen wurden einzeln je zweimal mit Ether extrahiert. Die Etherphasen wurden vereinigt, über Maguesiumsulfat getrocknet, filtriert und das Filtrat eingedampft. Das braune Rohprodukt wurde an 100 g Kieselgel mit Cyclohexan gereinigt. Die Produkt-haltigen Fraktionen wurden eingedampft und der Rückstand aus Isopropanol umkristallisiert. Dies ergab 0,57 g weisses 1-Fluor-4-[trans-4-(2,6-difluor-4-propylphenyläthinyl)-cyclohexyl]cyclohex-1-en, Smp. (C/N) 74,2°C, Klp. (N/I) 100,7°C.

Auf analoge Weise können folgende Verbindungen hergestellt werden:
1-Fluor-4-[trans-4-(2,6-difluor-4-ethylphenyläthinyl)cyclohexyl]cyclohex-1-en;
1-Fluor-4-[trans-4-(2,6-difluor-4-butylphenyläthinyl)cyclohexyl]cyclohex-1-en;
1-Fluor-4-[trans-4-(2,6-difluor-4-pentylphenyläthinyl)cyclohexyl]cyclohex-1-en;
1-Fluor-4-[2,6-difluor-4-(4-trans-ethylcyclohexyl)phenyläthinyl]cyclohex-1-en;
1-Fluor-4-[2,6-difluor-4-(4-trans-propylcyclohexyl)phenyläthinyl]cyclohex-1-en;
1-Fluor-4-[2,6-difluor-4-(4-trans-(E)-propenylcyclohexyl)phenyläthinyl]cyclohex-1-en;
1-Fluor-4-[2,6-difluor-4-(5-trans-propyl-1,3-dioxan-2-yl)phenyläthinyl] cyclohex-1-en;
1-Fluor-4-[2,6-difluor-4-(5-trans-pentyl-1,3-dioxan-2-yl)phenyläthinyl] cyclohex-1-en;
1-Fluor-4-[2,6-difluor-4-(5-trans-(E)-propenyl-1,3-dioxan-2-yl)phenyläthinyl]-cyclohex-1-en;
1-Fluor-4-{2,6-difluor-4-[5-trans-(3-butenyl)-1,3-dioxan-2-yl]phenyläthinyl}-cyclohex-1-en.

### Beispiel 3

a) Eine Lösung von 0,5 g 2,6-Difluor-4-propylbenzol in 5 ml Tetrahydrofuran wurde auf -70° gekühlt und innert 20 Minuten mit 2,2 ml 1,6 M Butyllithium versetzt. Nach 1 Stunde bei -70° wurde eine Lösung von 0,5 g 1,4-Dioxa-spiro[4,5]decan-8-on in 5 ml Tetrahydrofuran innert 15 Minuten zugetropft. Das Reaktionsgemisch wurde langsam auf Raumtemperatur erwärmt und eine weitere Stunde gerührt. Die Lösung wurde anschliessend mit 10 ml 10 proz. Essigsäure versetzt und zwischen Wasser und Ethylacetat verteilt. Die organische Phase wurde einmal mit Natriumcarbonatlösung und einmal mit Wasser gewaschen. Die wässrigen Phasen wurden einzeln je zweimal mit Ethylacetat extrahiert. Die organischen Phasen wurden vereinigt, über Maguesiumsulfat getrocknet, filtriert und das Filtrat vollständig eingedampft. Dies ergab 1,0 g rohes 8-(2,6-Difluor-4-propylphenyl)-8-hydroxy-1,4-dioxa-spiro[4,5]decan
b) Eine Lösung von 1,0 g 8-(2,6-Difluor-4-propylphenyl)-8-hydroxy-1,4-dioxa-spiro[4,5]decan in 25 ml Methylenchlorid wurde auf 0° abgekühlt und mit 440 µl Bortrifluorid-etherat versetzt. Nach 1 1/2 Stunden Rühren bei 0° wurde die Reaktionslösung mit 25 ml Natriumcarbonatlösung versetzt und anschliessend zwischen Wasser und Methylenchlorid verteilt. Die organische Phase wurde zweimal mit Natriumchloridlösung gewaschen. Die wässrigen Phasen wurden einzeln je zweimal mit Methylenchlorid extrahiert. Die Methylenchloridphasen wurden vereinigt, über Magnesiumsulfat getrocknet, filtriert und das Filtrat vollständig eingedampft. Dies ergab 1,0 g 8-(2,6-Difluor-4-propylphenyl)-1,4-dioxa-spiro[4,5]dec-7-en als gelbes Oel.
c) Das Produkt der vorhergehenden Stufe, 0,970 g 8-(2,6-Difluor-4-propyl-phenyl)-1,4-dioxa-spiro[4,5]dec-7-en, wurde in 10 ml Ethylacetat gelöst und in Gegenwart von 0,200 g 5% Pt/C hydriert. Die Hydrierung war nach 5 Stunden abgeschlossen. Danach wurde die Suspension filtriert, das Filtrat wurde vollständig eingedampft. Dies ergab 0,740 g 8-(2,6-Difluor-4-propyl-phenyl)-1,4-dioxa-spiro[4,5]decan als weisse feste Substanz.
d) Das hydrierte Produkt der vorhergehenden Stufe, 0,74 g 8-(2,6-Difluor-4-propylphenyl)-1,4-dioxa-spiro[4,5]decan, wurde in 4 ml Ameisensäure 98%ig und 8 ml Toluol aufgenommen und 1 Stunde gerührt, danach wurde die Reaktionslösung zwischen Wasser und Ether verteilt. Die organische Phase wurde einmal mit Wasser und einmal mit Natriumcarbonatlösung gewaschen. Die wässrigen Phasen wurden einzeln je zweimal mit Ether extrahiert. Die organischen Phasen wurden vereinigt, über Magnesiumsulfat getrocknet, filtriert und das Filtrat vollständig eingedampft. Dies ergab 0,68 g 4-(2,6-Difluor-4-propylphenyl)cyclohexanon.
e) Zu einer Lösung von 0,63 g 4-(2,6-Difluor-4-propylphenyl)cyclohexanon in 15 ml Dimethoxyethan und 50 µl Trifluormethansulfonsäure wurden in 3 Portionen über 60 Stunden 535 µl Diethylaminoschwefeltrifluorid (DAST) getropft. Die Reaktionslösung wurde anschliessend zwischen Natriumcarbonatlösung und Ether verteilt. Die organische Phase wurde zweimal mit Wasser gewaschen, und die wässrigen Phasen einzeln zweimal mit Ether extrahiert. Die organischen Phasen wurden vereinigt, über Magnesiumsulfat getrocknet, filtriert und das Filtrat vollständig eingedampft. Das erhaltene Rohprodukt (0,8 g) wurde chromatographisch gereinigt und ergab 0,36 g 1-Fluor-4-(2,6-difluor-4-propylphenyl)cyclohex-1-en, Smp. (C/I) 62,5°C.

Auf analoge Weise können folgende Verbindungen hergestellt werden:
1-Fluor-4-(2,6-difluor-4-ethylphenyl)cyclohex-1-en;
1-Fluor-4-(2,6-difluor-4-pentylphenyl)cyclohex-1-en;
1-Fluor-4-[2,6-difluor-4-(4-trans-propylcyclohexyl)phenyl]cyclohex-1-en;
1-Fluor-4-{2,6-difluor-4-[2-(4-trans-propylcyclohexyl)äthyl]phenyl}cyclohex-1-en;
1-Fluor-4-[2,6-difluor-4-(4-trans-butylcyclohexyl)phenyl]cyclohex-1-en, Smp. 75,5°C;
1-Fluor-4-[trans-4-(2,6-difluor-4-ethylphenyl)cyclohexyl]cyclohex-1-en;
1-Fluor-4-[trans-4-(2,6-difluor-4-propylphenyl)cyclohexyl]cyclohex-1-en;
1-Fluor-4-[trans-4-(2,6-difluor-4-pentylphenyl)cyclohexyl]cyclohex-1-en;
1-Fluor-4-{2-[trans-4-(2,6-difluor-4-propylphenyl)cyclohexyl]äthyl}cyclohex-1-en.

### Beispiel 4

a) 2,01 g 8-[trans-4-(2,6-Difluoro-4-propylphenyläthinyl)cyclohexyl]-1,4-dioxa-spiro[4,5]decan werden in 30 ml Essigester gelöst, mit 0,2 g 10% Palladium auf Kohle versetzt und bis zur Aufnahme von 0,01 Mol Wasserstoff bei Raumtemperatur und unter Normaldruck hydriert. Anschliessend wird über Celite filtriert und die klare Lösung eingedampft. Dies ergibt 8-{trans-4-[2-(2,6-Difluoro-4-propylphenyl)äthyl]cyclohexyl}-1,4-dioxa-spiro[4,5]decan.
b) 2 g 8-{trans-4-[2-(2,6-Difluoro-4-propylphenyl)äthyl]cyclohexyl}-1,4-dioxa-spiro[4,5]decan aus der vorherigen Stufe werden in 12 ml Ameisensäure 98%ig und 24 ml Toluol aufgenommen und 1 Stunde gerührt, danach wird die Reaktionslösung zwischen Wasser/Ether verteilt. Die organische Phase wird einmal mit Wasser und einmal mit Natriumcarbonatlösung gewaschen. Die wässrigen Phasen werden einzeln je zweimal mit Ether extrahiert. Die organischen Phasen werden vereinigt, über Magnesiumsulfat getrocknet, filtriert und das Filtrat vollständig eingedampft. Dies ergibt 4-{trans-4-[2-(2,6-Difluoro-4-propylphenyl)äthyl]cyclohexyl}cyclohexanon.
c) Zu einer Lösung von 1,7 g 4-{trans-4-[2-(2,6-Difluoro-4-propylphenyl)-äthyl]cyclohexyl}cyclohexanon, in 30 ml Dimethoxyethan und 0,1 ml Trifluormethansulfonsäure werden in 3 Portionen über 60 Stunden 1,1 ml Diethylaminoschwefeltrifluorid (DAST) getropft. Die Reaktionslösung wird anschliessend zwischen Natriumcarbonatlösung/Ether verteilt. Die organische Phase wird zweimal mit Wasser gewaschen, und die wässrigen Phasen einzeln zweimal mit Ether extrahiert. Die organischen Phasen werden vereinigt, über Magnesiumsulfat getrocknet, filtriert und das Filtrat vollständig eingedampft. Das erhaltene Rohprodukt wird über Kieselgel chromatographiert und ergibt 1-Fluor-4-{trans-4-[2-(2,6-Difluoro-4-propylphenyl)äthyl]cyclohexyl}cyclohex-1-en.

Auf analoge Weise können hergestellt werden:
1-Fluor-4-{trans-4-[2-(2,6-difluor-4-ethylphenyl)äthyl] cyclohexyl}cyclohex-1-en;
1-Fluor-4-{trans-4-[2-(2,6-difluor-4-pentylphenyl)äthyl]cyclohexyl}cyclohex-1-en;
1-Fluor-4-{2-[2,6-difluor-4-(trans-4-ethylcyclohexyl)phenyl]äthyl}cyclohex-1-en;
1-Fluor-4-{2-[2,6-difluor-4-(trans-4-propylcyclohexyl)phenyl]äthyl}cyclohex-1-en;
1-Fluor-4-{2-[2,6-difluor-4-(trans-4-pentylcyclohexyl)phenyl]äthyl}cyclohex-1-en.

### Beispiel 5

a) Zu einer Lösung von 0,303 g Lithiumaluminiumhydrid in 15 ml trockenem Tetrahydrofuran werden bei 0°C eine Lösung von 1,2 g 1-Fluor-4-(trans-4-methoxycarbonylcyclohexyl)cyclohex-1-en in 5 ml trockenem Tetrahydrofuran getropft. Dann wird eine Stunde gerührt, 1 ml Essigester zugefügt, 30 Minuten weiter gerührt und anschliessend mit 1n Salzsäure angesäuert. Dann wird mit 100 ml Wasser verdünnt, zweimal mit Methylenchlorid extrahiert und die vereinigten organischen Phasen mit Natriumbicarbonatlösung und mit Wasser gewaschen. Die organische Phase wird über Magnesiumsulfat getrocknet, filtriert, das Filtrat eingedampft und der Rückstand an Kieselgel chromatographiert. Dies ergibt 1-Fluor-4-(trans-4-hydroxymethylcyclohexyl)cyclohex-1-en.
b) Zu einer Lösung von 0,95 g 1-Fluor-4-(trans-4-hydroxymethylcyclohexyl)cyclohex-1-en in 10 ml Acetonitril werden bei Raumtemperatur 2,11 g Bromtriphenylphosphoniumbromid zugefügt und hierauf während 30 Minuten gerührt. Dann wird das Reaktionsgemisch vollständig eingedampft, der Rückstand in wenig Methylenchlorid gelöst und diese Lösung mit der zehnfachen Menge Pentan verdünnt. Die entstandene Suspension wird über Kieselgel/Celite filtriert und die Lösung vollständig eingedampft. Dies ergibt 1-Fluor-4-(trans-4-brommethylcyclohexyl)cyclohex-1-en.
c) Zu einer Lösung von 1,1 g 1-Fluor-4-(trans-4-brommethylcyclohexyl)cyclohex-1-en in 20 ml Aceton wird 1 g fein gemahlenes Kaliumcarbonat und eine Lösung von 0,7 g 2,6-Difluor-4-propylphenol in 5 ml Aceton gefügt und bei Rückflusstemperatur während 15 Stunden gerührt. Danach wird das Lösungsmittel eingeengt, der Rückstand zwischen Wasser und Methylenchlorid verteilt und die organische Phase über Magnesiumsulfat getrocknet. Dann wird die Lösung filtriert, das Filtrat vollständig eingedampft und der Rückstand an Kieselgel chromatographiert. Dies ergibt 1-Fluor-4-[trans-4-(2,6-difluor-4-propylphenoxymethyl)cyclohexyl]cyclohex-1-en

Auf analoge Weise können folgende Verbindungen hergestellt werden:
1-Fluor-4-[trans-4-(2,6-difluor-4-ethylphenoxymethyl)cyclohexyl]cyclohex-1-en;
1-Fluor-4-[trans-4-(2,6-difluor-4-butylphenoxymethyl)cyclohexyl]cyclohex-1-en;
1-Fluor-4-[trans-4-(2,6-difluor-4-pentylphenoxymethyl)cyclohexyl]cyclohex-1-en;
1-Fluor-4-[2,6-difluor-4-(trans-4-propylcyclohexyl)phenoxymethyl]cyclohex-1-en;
1-Fluor-4-[2,6-difluor-4-(trans-4-pentylcyclohexyl)phenoxymethyl]cyclohex-1-en;
1-Fluor-4-[2,6-difluor-4-(trans-4-vinylcyclohexyl)phenoxymethyl]cyclohex-1-en;
1-Fluor-4-[2,6-difluor-4-(trans-4-(E)-propenylcyclohexyl)phenoxymethyl]cyclohex-1-en;
1-Fluor-4-{2,6-difluor-4-[trans-4-(3-butenyl)cyclohexyl]phenoxymethyl}cyclohex-1-en.

### Beispiel 6

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung und die Ansprechzeiten wurden in einer TN-Zelle (low bias tilt) mit 8 µm Plat-tenabstand bei 22°C gemessen; als Betriebsspannung wurde der 2,5-fache Wert der Schwellenspannung (V₁₀) gewählt. Die entsprechenden Daten für 4-(trans-4-Pentyl-cyclohexyl)benzonitril betragen: Klp. (N-I) = 54.6°C, V₁₀ = 1,62 V, tₒₙ = 22ms, t_{off} = 42 ms, Δn = 0,120.

### BM-1

- 90 Gew.%: 4-(trans-4-Pentyl-cyclohexyl)benzonitril
- 10 Gew.%: 1-Fluor-4-[trans-4-(2,6-difluor-4-propylphenyläthinyl)cyclohexyl]cyclohex-1-en
Klp. (N/I): 55,0°C, V₁₀ = 1,53 V, tₒₙ = 29 ms, t_{off} = 46 ms, Δn = 0,123

### BM-2

- 80 Gew.%: 4-(trans-4-Pentyl-cyclohexyl)benzonitril
- 20 Gew.%: 1-Fluor-4-[trans-4-(2,6-difluor-4-propylphenyläthinyl)cyclohexyl]cyclohex-1-en
Klp. (N/I): 56,1°C, V₁₀ = 1,47 V, tₒₙ = 33 ms, t_{off} = 52 ms, Δn = 0,121

### BM-3

- 90 Gew.%: 4-(trans-4-Pentyl-cyclohexyl)benzonitril
- 10 Gew.%: trans-4-(1-Fluorcyclohex-1-en-4-yl)cylohexanoyl-2,6-difluor-4-propylphenolat
Klp. (N/I): 54,1°C, V₁₀ = 1,46 V, tₒₙ = 36 ms, t_{off} = 48 ms, Δn = 0,116

### BM-4

- 80 Gew.%: 4-(trans-4-Pentyl-cyclohexyl)benzonitril
- 20 Gew.%: trans-4-(1-Fluorcyclohex-1-en-4-yl)cylohexanoyl-2,6-difluor-4-propylphenolat
Klp. (N/I): 55,7°C, V₁₀ = 1,38 V, tₒₙ = 43 ms, t_{off} = 59 ms, Δn = 0,111

### BM-5

- 90 Gew.%: 4-(trans-4-Pentyl-cyclohexyl)benzonitril
- 10 Gew.%: 1-Fluor-4-[2,6-difluor-4-(4-trans-butylcyclohexyl)phenyl]-cyclohex-1-en
Klp. (N/I): 47,9°C, V₁₀ = 1,45 V, tₒₙ = 31 ms, t_{off} = 51 ms, Δn = 0,114

### BM-6

- 80 Gew.%: 4-(trans-4-Pentyl-cyclohexyl)benzonitril
- 20 Gew.%: 1-Fluor-4-[2,6-difluor-4-(4-trans-butylcyclohexyl)phenyl]-cyclohex-1-en
Klp. (N/I): 40,9°C, V₁₀ = 1,29 V, tₒₙ = 42 ms, t_{off} = 69 ms, Δn = 0,103

## Patentansprüche

1. Verbindungen der allgemeinen Formel 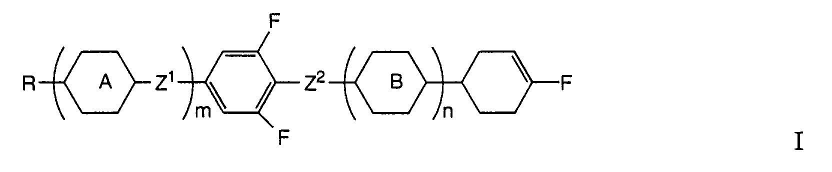 worin
R Alkyl oder Alkenyl mit höchstens 12 Kohlenstoffatomen;
Ringe A und B unabhängig voneinander 1,4-Phenylen, trans-1,4-Cyclohexylen oder trans-1,3-Dioxan-2,5-diyl;
Z¹ eine Einfachbindung oder -CH₂CH₂-; und
Z² eine Einfachbindung, -CH₂CH₂-, -OCH₂-, -C≡C-, -COO-oder -OOC- bedeuten; und
m, n 0 oder 1 sind, mit der Massgabe, dass m + n ≦ 1 ist.

2. Verbindungen gemäss Anspruch 1, der Formeln 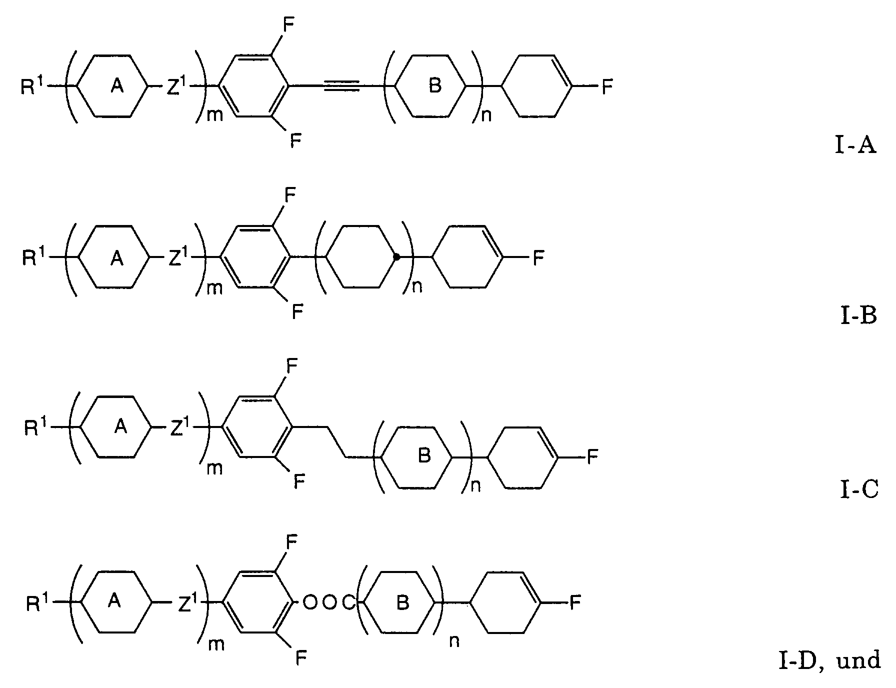 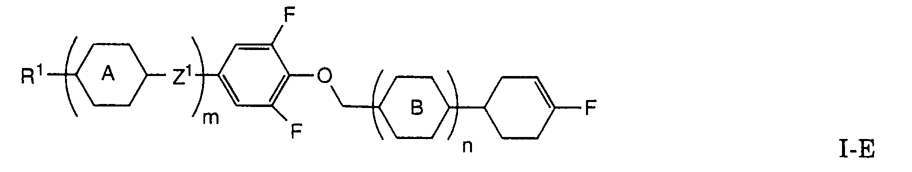 worin R¹ Alkyl, 3E-Alkenyl oder an einem gesättigten Ring auch Vinyl oder 1E-Alkenyl mit je höchstens 7 Kohlenstoffatomen bedeutet.

3. Verbindungen gemäss Anspruch 2, der Formeln 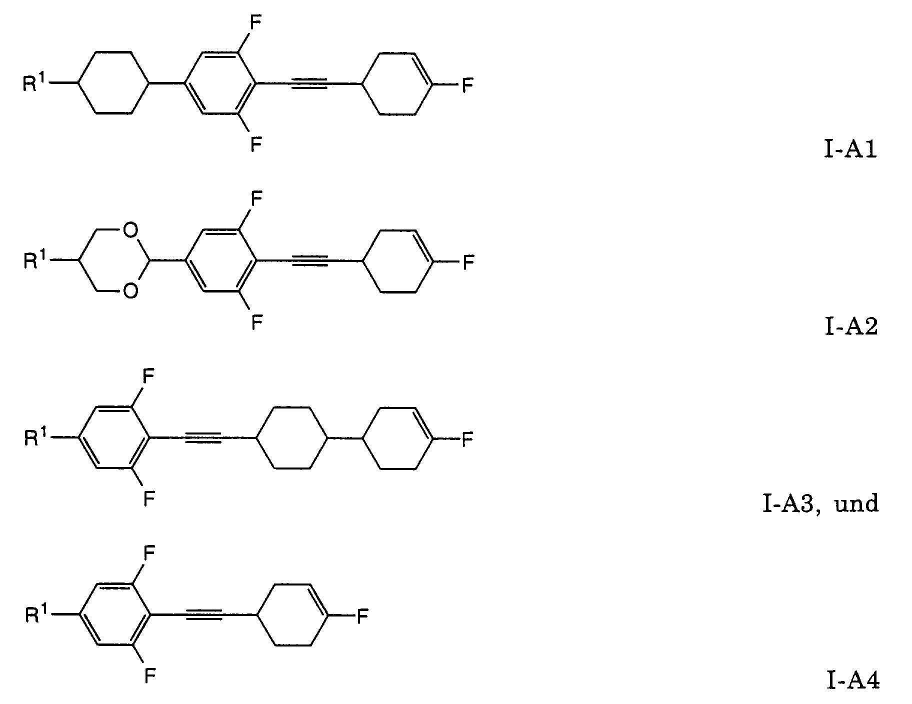 worin R¹ Alkyl, 3E-Alkenyl oder an einem gesättigten Ring auch Vinyl oder 1E-Alkenyl mit je höchstens 7 Kohlenstoffatomen bedeutet.

4. Verbindungen nach Anspruch 2, der Formeln 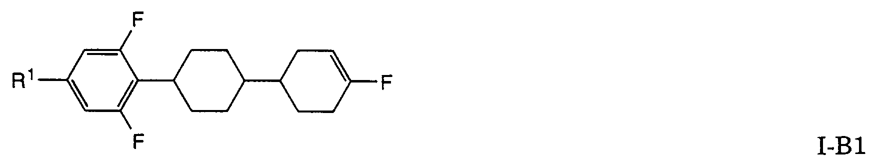 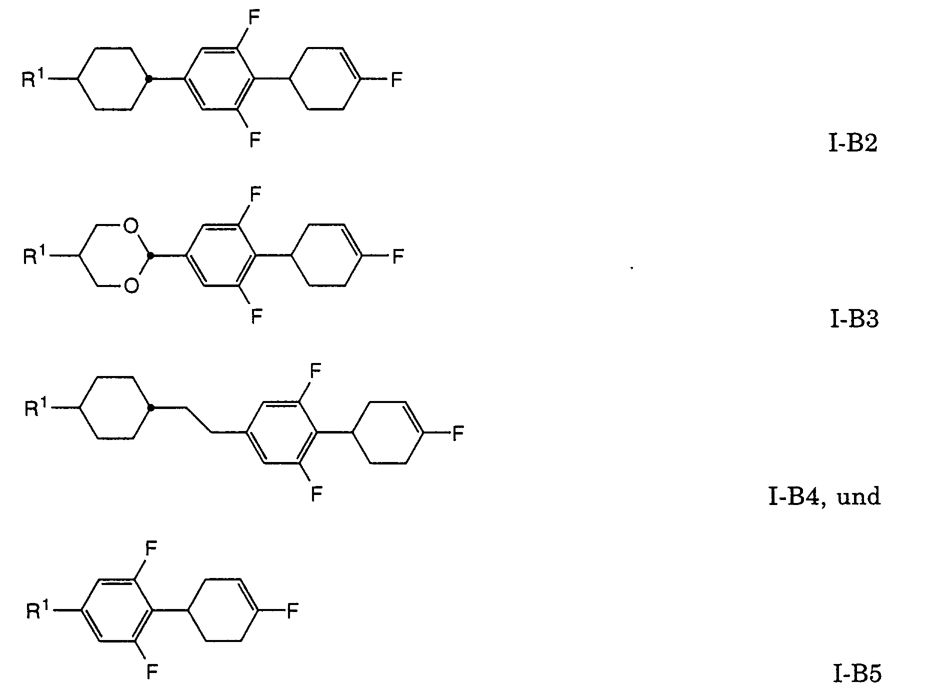 worin R¹ Alkyl, 3E-Alkenyl oder an einem gesättigten Ring auch Vinyl oder 1E-Alkenyl mit je höchstens 7 Kohlenstoffatomen bedeutet.

5. Verbindungen nach Anspruch 2, der Formeln 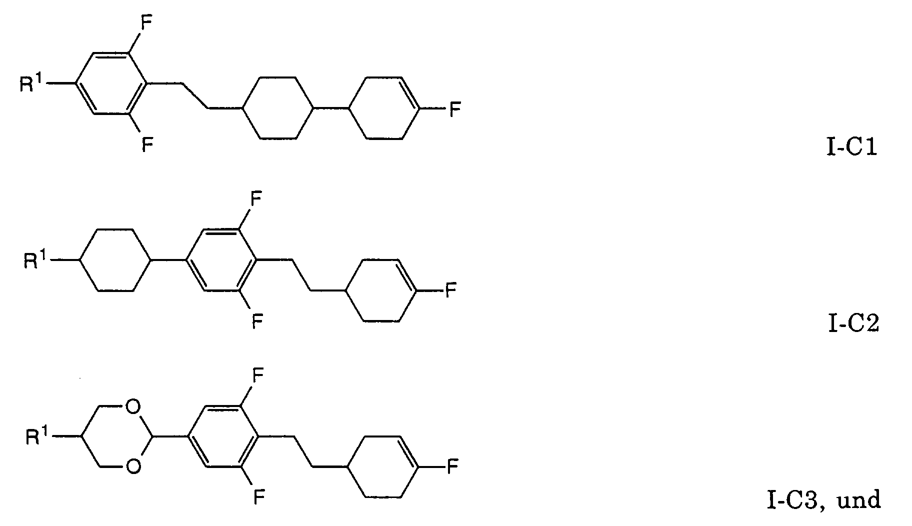 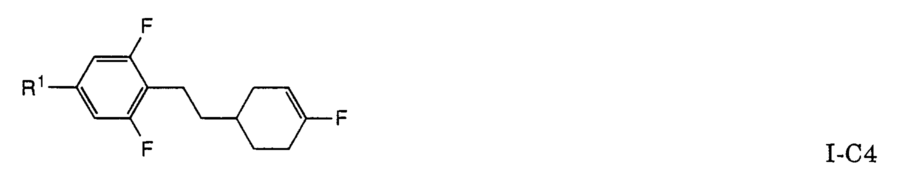 worin R¹ Alkyl, 3E-Alkenyl oder an einem gesättigten Ring auch Vinyl oder 1E-Alkenyl mit je höchstens 7 Kohlenstoffatomen bedeutet.

6. Verbindungen nach Anspruch 2, der Formeln 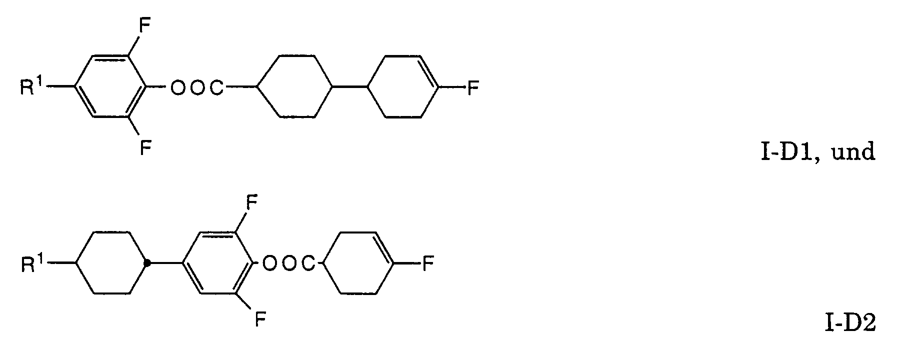 worin R¹ Alkyl, 3E-Alkenyl oder an einem gesättigten Ring auch Vinyl oder 1E-Alkenyl mit je höchstens 7 Kohlenstoffatomen bedeutet.

7. Verbindungen nach Anspruch 2, der Formeln 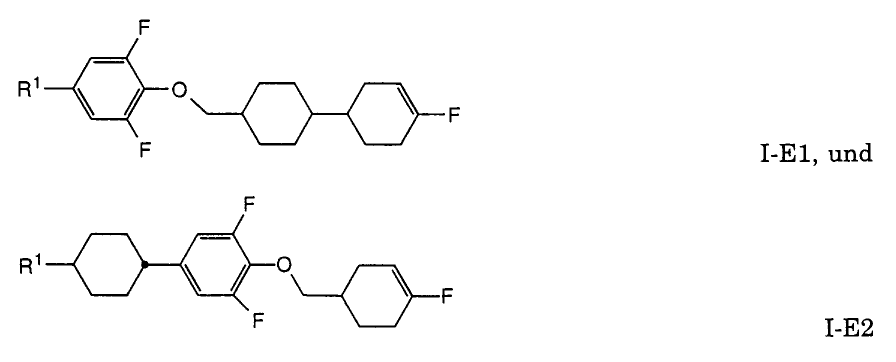 worin R¹ Alkyl, 3E-Alkenyl oder an einem gesättigten Ring auch Vinyl oder 1E-Alkenyl mit je höchstens 7 Kohlenstoffatomen bedeutet.

8. Verbindungen nach einem der Ansprüche 1 bis 7, dadurch gekennzeichnet, dass der Rest R¹ Ethyl, Propyl, Butyl, Pentyl, 3-Butenyl oder 3E-Pentenyl und an trans-1,4-Cyclohexylen oder trans-1,3-Dioxan-2,5-diyl auch Vinyl, 1E-Propenyl, 1E-Butenyl oder 1E-Pentenyl bedeutet.

9. Die Verbindung nach Anspruch 2 und 3, 1-Fluor-4-[trans-4-(2,6-difluor-4-propylphenyläthinyl)cyclohexyl]cyclohex-1-en.

10. Die Verbindung nach Anspruch 2 und 4, 1-Fluor-4-(2,6-difluor-4-propylphenyl)cyclohex-1-en.

11. Die Verbindung nach Anspruch 2 und 6, trans-4-(1-Fluorcyclohex-1-en-4-yl)cyclohexanoyl-2,6-difluor-4-propylphenolat.

12. Flüssigkristallines Gemisch mit mindestens 2 Komponenten, dadurch gekennzeichnet, dass mindestens eine Komponente eine Verbindung der in Anspruch 1 definierten Formel I ist.

13. Flüssigkristallines Gemisch nach Anspruch 12, dadurch gekennzeichnet, dass der Anteil an Verbindungen der Formel I 1-70 Gew.-% beträgt.

14. Flüssigkristallines Gemisch nach Anspruch 12 oder 13, dadurch gekenzeichnet, dass der Anteil an Verbindungen der Formel I 5-20 Gew.-% beträgt.

15. Verwendung der Verbindungen der in Anspruch 1 definierten Formel I für elektro-optische Vorrichtungen.

16. Verwendung von flüssigkristallinen Mischungen nach einem der Ansprüche 12 bis 14 für elektro-optische Vorrichtungen.
